# FASCICULE DE BREVET EUROPEEN

(11) **EP 2 331 702 B1**
(45) Date de publication et mention de la délivrance du brevet: **06.11.2013**
(21) Numéro de dépôt: 09740462.8
(22) Date de dépôt: 13.08.2009
(51) Int. Cl.: C12Q 1/14, C12R 1/445

(54) **MILIEU DE CULTURE PERMETTANT DE DIFFERENCIER STAPHYLOCOCCUS AUREUS DES STAPHYLOCOCCUS A COAGULASE NEGATIVE**
KULTURMEDIUM ZUR UNTERSCHEIDUNG ZWISCHEN STAPHYLOCOCCUS AUREUS UND COAGULASE-NEGATIVEN STAPHYLOKOKKEN
CULTURE MEDIUM ENABLING THE DIFFERENTIATION OF STAPHYLOCOCCUS AUREUS FROM COAGULASE-NEGATIVE STAPHYLOCOCCI

(30) Priorité: 13.08.2008 FR 0855555
(43) Date de publication de la demande: 15.06.2011
(73) Titulaire: Biomérieux, 69280 Marcy L'etoile (FR)
(72) Inventeur: MOSTICONE, David, F-69280 Sainte Consorce (FR); ORENGA, Sylvain, F-01160 Neuville Sur Ain (FR); VIMONT, Antoine, F-69005 Lyon (FR); GUO, Yuping, F-69160 Tassin La Demi Lune (FR); DHEDIN, Martine, F-69210 Sain Bel (FR)
(86) Numéro de dépôt international: PCT/FR2009/051588
(87) Numéro de publication internationale: WO 2010/018349

(56) Documents cités:
- EP-A- 1 219 628
- WO-A1-00/53799
- MARQUES M B ET AL.: "Growth in acidic media increases production of phosphatidylinositol- specific phospholipase C by Staphylococcus aureus" CURRENT MICROBIOLOGY, vol. 25, no. 3, 1992, pages 125-128, XP009115720 NEW YORK, US ISSN: 0343-8651 DOI: 10.1007/BF01571019
- DAUGHERTY S ET AL.: "Cloning, expression, and mutagenesis of phosphatidylinositol-specific phospholipase C from Staphylococcus aureus: A potential staphylococcal virulence factor" INFECTION AND IMMUNITY, vol. 61, no. 12, 1993, pages 5078-5089, XP009115687 ISSN: 0019-9567 cité dans la demande
- RYAN M ET AL.: "A chemiluminescent substrate for the detection of phosphatidylinositol-specific phospholipase C" ANALYTICAL BIOCHEMISTRY, vol. 214, no. 2, 1 novembre 1993 (1993-11-01), pages 548-556, XP24763481 UNITED STATES ISSN: 0003-2697 DOI: 10.1006/abio.1993.1537
- RUKAVISHNIKOV A V ET AL.: "Improved synthesis of myo-inositol 1-(4-nitrophenyl hydrogen phosphate), a chromogenic substrate for phosphatidylinositol-specific phospholipase C" CHEMISTRY AND PHYSICS OF LIPIDS, vol. 89, no. 2, 22 octobre 1997 (1997-10-22), pages 153-157, XP9126202 Ireland ISSN: 0009-3084 DOI: 10.1016/S0009-3084(97)00069-8 cité dans la demande
- SHASHIDHAR M S ET AL.: "A chromogenic substrate for phosphatidylinositol-specific phospholipase C: 4-nitrophenyl myo-inositol-1-phosphate." CHEMISTRY AND PHYSICS OF LIPIDS, vol. 60, no. 2, décembre 1991 (1991-12), pages 101-110, XP23389725 Ireland ISSN: 0009-3084 DOI: 10.1016/0009-3084(91)90032-7
- MARQUES M B ET AL.: "Phosphatidylinositol-specific phospholipase C, a possible virulence factor of Staphylococcus aureus" JOURNAL OF CLINICAL MICROBIOLOGY, vol. 27, no. 11, novembre 1989 (1989-11), pages 2451-2454, XP9115722 UNITED STATES ISSN: 0095-1137
- ORENGA S ET AL.: "Enzymatic substrates in microbiology." JOURNAL OF MICROBIOLOGICAL METHODS, vol. 79, no. 2, 11 août 2009 (2009-08-11), - novembre 2009 (2009-11) pages 139-155, XP26696833 Netherlands ISSN: 1872-8359 DOI: 10.1016/j.mimet.2009.08.001

## Description

La présente invention concerne, de façon générale, le domaine de l'analyse microbiologique. Plus particulièrement, la présente invention concerne un milieu de culture sélectif pour la croissance, la détection, l'identification et/ou le dénombrement des staphylocoques, permettant de différencier *Staphylococcus aureus* des staphylocoques à coagulase négative. L'invention est définie par les revendications.

Les bactéries du genre *Staphylococcus* ou staphylocoques sont responsables d'un grand nombre d'infections nosocomiales et représentent un problème hospitalier important. Ces bactéries sont des *cocci* à Gram positif, qui peuvent être classées en deux grands groupes que l'on distingue par la production d'une protéine, la coagulase, déclenchant la coagulation du plasma. On distingue ainsi les staphylocoques à coagulase négative, dont le représentant principal est *Staphylococcus epidermidis* et les staphylocoques à coagulase positive, dont le représentant principal est *Staphylococcus aureus,* bien connu pour sa virulence. Les staphylocoques sont largement répandus dans l'environnement, la peau et les muqueuses de l'homme et de l'animal. La desquamation régulière de ces hôtes a pour effet de les disperser abondamment dans la nature (eau, sol, air, aliments, objets), et en milieu hospitalier, des mesures draconiennes d'hygiène et d'isolement des patients sont requises pour limiter la dissémination des souches épidermiques. Ainsi, *Staphylococcus aureus,* qui représente 80 à 90% des staphylocoques isolés en clinique est un pathogène majeur de l'homme responsable de nombreuses infections en milieu hospitalier, telles que notamment lors de pneumonies nosocomiales, d'infections de plaies chirurgicales, d'infections de brûlures, d'infections des corps étrangers (valves cardiaques, prothèses de hanche, agrafes...) et d'infections systémiques ou de septicémies qui sont souvent dues à l'usage de cathéters intra vasculaires, ou à la dissémination de la bactérie à partir d'un autre foyer infectieux.

Dans le domaine alimentaire, le risque constitué par les staphylocoques, et en particulier par *Staphylococcus aureus,* est lui aussi très important. En effet, certaines souches de *Staphylococcus aureus* sont capables de produire des entérotoxines, dont l'ingestion par le consommateur entraîne une intoxication, source de nausées, douleurs abdominales et surtout vomissements violents et répétés souvent accompagnés de diarrhée.

Les toxi-infections alimentaires à staphylocoques représentent ainsi une des premières toxi-infections alimentaires d'origine bactérienne.

La dissémination se produit généralement par les animaux et l'homme qu'ils soient malades ou porteurs sains, par le lait cru (mammites), par l'air et les surfaces ou le matériel contaminés au contact d'aliments et de porteurs sains ou infectés.

La détection et l'identification dans les aliments des staphylocoques et en particulier de *Staphylococcus aureus,* constituent donc un enjeu de santé publique majeur.

Parmi les milieux de culture utilisés pour la détection, l'identification et le dénombrement des bactéries du genre *Staphylococcus,* on distingue les milieux non sélectifs tels le milieu Columbia ou la gélose trypticase-soja et les milieux sélectifs, tels la gélose Chapman et la gélose Baird-Parker. *Staphylococcus aureus* peut aussi être détecté sur une gélose au sang en fonction de ses caractéristiques morphologiques et de son profil d'hémolyse mais cette méthode est peu sensible et spécifique et n'est que peu ou pas utilisée dans l'industrie agro-alimentaire. Le bouillon coeur-cervelle est également employé de façon usuelle pour la recherche de staphylocoques.

Les milieux de culture bactériologiques spécifiques favorisent la croissance de certains microorganismes et limitent celle des autres. Ils contiennent au moins un agent inhibiteur de microorganismes autres que le pathogène cible. L'effet des inhibiteurs doit rester limité sur le microorganisme d'intérêt, d'autant que le dit microorganisme d'intérêt peut être endommagé dans les préparations alimentaires élaborées. La gélose Chapman correspond à un milieu hyper salin sur une base nutritive ordinaire, utilisant le mannitol comme substrat dont la fermentation est révélée par un indicateur de pH. Ledit indicateur de pH peut être un indicateur coloré ou un indicateur fluorescent. Le milieu de Baird-Parker correspond à une base nutritive riche additionnée de tellurite de potassium et de chlorure de lithium, agents sélectifs communément utilisés pour la croissance de *Staphylococcus aureus.* Le chlorure de lithium est un inhibiteur des entérocoques. D'autres éléments chimiques peuvent être combinés avec le tellurite de potassium et le chlorure de sodium ou de lithium : le sulfate d'ammonium, l'acide sorbique, la glycine, la polymyxine B.

Néanmoins, l'analyse des matrices alimentaires pour les staphylocoques potentiellement toxinogènes présente des problèmes spéciaux qui limitent l'utilisation pratique des méthodes développées pour la clinique. En effet, les milieux couramment proposés permettent aussi la croissance et, selon le caractère phénotypique recherché, la détection des staphylocoques à coagulase négative qui ne sont pas considérés comme des agents de contamination alimentaire.

Dans le cas du milieu Baird-Parker + RPF (plasma de lapin + fibrinogène bovin), qui est le milieu de référence conformément à la norme ISO 6888-1 et 6888-2, on observe généralement un certain nombre d'inconvénients.

En premier lieu, il existe des difficultés de lecture et de sensibilité : de faux négatifs peuvent potentiellement apparaître du fait que certaines souches de *Staphylococcus aureus* ne se développent pas sur ce milieu ou présentent une coagulase très faible et tardive faisant croire à la présence de staphylocoques à coagulase négative. D'autre part, des résultats faussement négatifs peuvent être également observés en raison d'interférences matricielles : en effet pour le dénombrement de faibles niveaux de contamination de *Staphylococcus aureus,* une dilution minimale de l'échantillon est effectuée (1/10) ce qui engendre une difficulté de lecture du halo due à la présence de composés matriciels (e.g échantillons de lait et/ou produits laitiers : couleur blanche de la caséine masquant le halo révélant la coagulase).

Le milieu Baird-Parker + RPF nécessite la combinaison d'une source de thrombine et d'une source de plasminogène. Ceux-ci sont obtenus à partir du sang d'animaux, ce qui pose des problèmes de fiabilité d'approvisionnement (qualité, quantité, ...).

Par ailleurs, la lecture d'un halo n'est pas possible en bouillon (milieu liquide) et le contraste entre le halo et le milieu peut être réduit. Enfin en cas de colonies confluentes, il est difficile de déterminer celles qui produisent le halo de celles qui ne le produisent pas.

En second lieu, il peut également exister des problèmes de spécificité : en présence d'une flore annexe abondante (comme par exemple *Bacillus spp),* des résultats faussement positifs peuvent apparaître avec ce type de milieu. C'est par exemple le cas pour le dénombrement de *Staphylococcus aureus* dans certains produits carnés (saucissons secs) et laitiers (fromages type Munster) dans lesquels *Staphylococcus xylosus* est utilisé comme ferment. Ainsi, des colonies noires entourées d'un halo apparaissent sur le milieu Baird Parker + RPF, colonies normalement caractéristiques de *Staphylococcus aureus* sur ce milieu, mais qui sont en réalité consécutives à une croissance contiguë de souches de *Staphylococcus xylosus* (produisant des colonies noires sans halo) et de certaines souches de *Bacillus* générant un trouble pouvant être identifié comme un halo sur la gélose.

WO 00/53799 décrit un milieu pour détecter S. aureus grâce à deux agents chromogènes.

Parmi les méthodes alternatives, telles que les milieux chromogènes, compte tenu des similitudes accrues entre les staphylocoques à coagulase positive et les staphylocoques à coagulase négative, il n'existe aucun milieu permettant de discriminer très spécifiquement ces deux groupes. En effet, les concepts de différentiation (i.e. substrats et/ou sucres plus ou moins spécifiques associé(s) à un système inhibiteur) mis en oeuvre dans ce type de milieux dans le but de différencier les staphylocoques à coagulase positive des staphylocoques à coagulase négative, sont très souvent complexes et peu discriminants, notamment pour les prélèvements alimentaires contenant une flore microbienne variée et abondante.

L'absence de substrats ou sucres spécifiques pour la différentiation entre les staphylocoques à coagulase positive et les staphylocoques à coagulase négative (voire d'autres espèces bactériennes) implique l'utilisation de systèmes sélectifs/inhibiteurs « agressifs » qui peuvent altérer ou inhiber la croissance ou l'activité enzymatique considérée des *Staphylococcus aureus,* conduisant alors à l'obtention potentielle de résultats faussement négatifs.

Des résultats faussement négatifs en cas de faibles contaminations peuvent également être obtenus avec certains milieux, en raison des dilutions nécessaires pour s'affranchir des interactions matricielles : e.g coloration rose provoquée par des niveaux élevés de phosphatase présente dans les produits laitiers, avec 3M™ Petrifilm™ Staph Express Count Plates.

A l'inverse, les milieux basés sur la dégradation d'un substrat ou la fermentation d'un sucre peu spécifique des *Staphylococcus aureus,* associé qui plus est à un système inhibiteur qui n'est pas assez sélectif, conduisent ainsi à l'obtention de résultats faussement positifs.

Enfin, certains milieux sont basés sur l'utilisation de deux substrats afin d'assurer une différenciation optimale entre *Staphylococcus aureus* et les autres staphylocoques et espèces bactériennes. La mise en oeuvre d'un tel concept engendre un coût supérieur du milieu sans permettre une spécificité suffisante, notamment dans le cas des prélèvements d'origine alimentaire.

Les enzymes de type phospholipase C sont connues et décrites dans la littérature comme étant présentes dans bon nombre de microorganismes.

Parmi les phospholipases C, la Phosphatidylinositol Phospholipase C (PIPLC) de *Staphylococcus aureus* a été purifiée et caractérisée (Phosphatidylinositol-Specific Phospholipase C from Staphylococcus aureus, METHODS IN ENZYMOLOGY, 1981 - Vol 71).

Cette enzyme a également été décrite comme étant un possible facteur de virulence de *Staphylococcus aureus* (JOURNAL OF CLINICAL MICROBIOLOGY, Nov. 1989, p. 2451-2454 - Vol. 27, No. 11 et INFECTION AND IMMUNITY, Dec. 1993, p. 5078-5089 - vol.61, N°12). Par ailleurs, des études tendant à évaluer les conditions *in vitro* permettant une production maximale de PIPLC active chez *Staphylococcus aureus,* ont également été publiées (Marques et al. ; Growth in Acidic Media Increases Production of Phosphatidylinositol-Specific Phospholipase C by Staphylococcus aureus ; CURRENT MICROBIOLOGY Vol. 25 (1992), pp.125-128).

RYAN M ET AL.: "A chemiluminescent substrate for the detection of phosphatidylinositol-specific phospholipase C", ANALYTICAL BIOCHEMISTRY, vol. 214, no. 2, novembre 1993, pages 548-556, décrit l'utilisation d'un substrat chimiluminescent (LUMI-PI) pour la détection de la PI-PLC, secrétée par S. aureus. Ce document indique que la PI-PLC est un facteur de virulence probable de S. aureus.

SHASHIDHAR M S ET AL.: "A chromogenic substrate for phosphatidylinositol-specific phospholipase C: 4-nitrophenyl myo-inositol-1-phosphate.", CHEMISTRY AND PHYSICS OF LIPIDS, vol. 60, no. 2, décembre 1991, pages 101-110, décrit le 4-nitrophényl myo-inositol-1-phosphate comme substrat chromogénique de la PI-PLC utilisé dans un test spectrophotométrique pour détecter des bactéries Bacillus cereus. D'autre part, la PI-PLC est décrite comme un facteur de virulence probable de S. aureus et Listeria monocytogenes.

Le document EP-0 970 239 B1 décrit de nouveaux substrats chromogènes permettant la détection de la PIPLC, secrétée par différents microorganismes. Les exemples cités décrivent, d'une part, la préparation des différents substrats évoqués et d'autre part, mettent en évidence les conditions optimales d'utilisation de la méthode (e.g concentration substrat/ enzyme, inducteurs recommandés...). En revanche, aucun milieu de culture permettant la détection des Staphylocoques n'est décrit dans ce document.

Le document EP-1 506 309 B1 décrit un milieu de culture pour la détection d'un microorganisme capable de produire une PIPLC, ledit milieu de culture contenant en combinaison, au moins un composé fluorogène et au moins un composé chromogène, capable respectivement de générer de la fluorescence et une coloration lorsqu'ils sont en contact avec la PIPLC. Le milieu de culture est décrit comme permettant sans ajout d'inhibiteur, la détection de différentes espèces bactériennes, telles que *Listeria monocytogenes, Listeria ivanovii, Bacillus cereus, Bacillus thuringiensis, Bacillus mycoides, Bacillus anthracis, Staphylococcus aureus, Legionella pneumophila,* des espèces de *Clostridium, Helicobacter pylori,* des espèces de *Candida* et des espèces de *Aspergillus.* Nonobstant, aucun milieu de culture en tant que tel, destiné à détecter *Staphylococcus aureus,* n'est décrit dans ce document. Les seuls milieux de culture décrits concernent la détection *Listeria monocytogenes* et *Bacillus* groupe *cereus.* Il apparaît de surcroît que sur tous le milieux de culture décrits, les espèces de staphylocoques et en particulier *Staphylococcus aureus* ne poussent pas.

Le document EP-0 949 266 B1 décrit des substrats spécifiques de la PIPLC en tant qu'indicateur d'activité bactérienne en particulier du genre *Listeria.* Ledit substrat contient au moins un composé apte à produire une couleur ou de la fluorescence. Les seuls milieux de culture décrits concernent la détection de *Listeria monocytogenes.* Il apparaît de surcroît que sur tous les milieux de culture décrits, les espèces de staphylocoques et en particulier *Staphylococcus aureus* ne poussent pas, du fait de leur inhibition.

Comme autre phospholipase C, la Phosphatidylcholine Phospholipase C (PCPLC) a également été décrite comme étant présente chez différentes bactéries, dont *Staphylococcus aureus* (J.G. Songer, Trends in Microbiology, Volume 5, Number 4, April 1997 , pp. 156-161(6)).

Le document EP-1 219 628 décrit de nouveaux substrats colorimétriques pour la détection et l'identification de microorganismes. Ces substrats sont des substrats spécifiques de PCPLC. Néanmoins, ce document ne décrit pas de substrats pour la détection et l'identification de *Staphylococcus aureus,* ni de milieu de culture *ad hoc.*

Ainsi, il apparaît que, malgré la caractérisation de la PIPLC de *Staphylococcus aureus* il y a de cela plus de 25 ans et l'identification d'un potentiel rôle dans la virulence de cette espèce bactérienne, il n'a jamais été décrit un milieu de culture spécifique pour la détection et/ou le dénombrement de *Staphylococcus aureus,* basé sur l'utilisation d'au moins un substrat spécifique de la PIPLC, chromogène, fluorogène ou luminescent, et permettant la discrimination entre cette espèce et les autres espèces de staphylocoques, l'inhibition des souches de *Staphylococcus aureus* sur les milieux décrits rendant *a priori* leur utilisation impossible pour une telle application. Par ailleurs, aucun document n'a décrit l'utilisation d'un substrat spécifique de PCPLC, chromogène, fluorogène ou luminescent, dans un milieu de culture spécifique pour la détection ou le dénombrement de *Staphylococcus aureus,* l'utilisation dudit substrat permettant la discrimination entre cette espèce et les autre espèces de staphylocoques.

Eu égard aux problèmes soulevés par l'état de la technique considéré ci-dessus, un des objectifs essentiels de la présente invention est de fournir un milieu de culture favorisant la croissance des staphylocoques dans le but de les détecter et/ou les identifier et/ou les dénombrer et permettant de discriminer *Staphylococcus aureus* des autres espèces de staphylocoques.

Un autre objectif de la présente invention est de fournir un milieu de culture limitant l'obtention de résultats faussement positifs, notamment dus aux staphylocoques à coagulase négative.

Un autre objectif de la présente invention est de fournir un milieu de culture permettant un meilleur recouvrement des bactéries cibles, notamment dans le cas de faibles niveaux de contamination, par l'utilisation d'un système inhibiteur réduit.

Un autre objectif de la présente invention est de fournir un milieu de culture permettant une lecture et une interprétation simple, grâce à l'utilisation d'un seul substrat spécifique.

Un autre objectif de la présente invention est de fournir un milieu de culture permettant une automatisation de la lecture.

Enfin, un dernier objectif de la présente invention est de fournir un milieu de culture permettant de réduire le temps de rendu des résultats, grâce à une sélectivité réduite favorisant la croissance des *Staphylococcus aureus.*

Ces objectifs, parmi d'autres, sont atteints par la présente invention qui concerne en premier lieu un milieu de culture spécifique pour la croissance des bactéries *Staphylococcus aureus* et leur détection et/ou identification, ledit milieu étant caractérisé par le fait qu'il comporte au moins un substrat chromogène de phosphatidyl inositol phospholipase C qui est le 4-Nitrophenyl myo-inositol-1-phosphate.

Par substrat, on entend toute molécule susceptible d'engendrer directement ou indirectement un signal détectable dû à une activité enzymatique ou métabolique du microorganisme.

Le substrat est un substrat enzymatique, c'est à dire un substrat pouvant être métabolisé par une enzyme en un produit permettant la détection, directe ou indirecte d'un microorganisme.

Ce substrat comprend notamment une première partie spécifique de l'activité enzymatique à révéler et une seconde partie faisant office de marqueur, ci-après appelée partie marqueur. Cette partie marqueur est chromogène.

Au sens de la présente invention, ce substrat est un substrat de la phosphatidyl inositol phospholipase C (PIPLC). La concentration en substrat de PIPLC dans le milieu peut être comprise entre 0,01 et 1 g/l.

Dans un mode de réalisation préférentiel, le milieu de culture selon l'invention se présente sous forme liquide. En effet, cette forme est particulièrement adaptée à l'analyse microbiologique alimentaire, qui peut nécessiter une phase de malaxage des échantillons solides dans le milieu de culture, pour permettre la libération des microorganismes potentiellement présents dans lesdits échantillons.

Néanmoins, le milieu de culture peut également se présenter sous forme solide (e.g. milieu gélosé à base d'agar). De façon identique aux milieux liquides, le substrat PIPLC peut être présent dans ces milieux solides et permettre la détection, l'identification, voire en outre le dénombrement de *Staphylococcus aureus.*

Alternativement, le milieu de culture selon l'invention peut comporter en outre un substrat permettant de révéler une activité enzymatique ou métabolique des microorganismes cibles, différente de l'activité phospholipase C, telle que l'activité estérase (notamment lipase ou phosphatase) coagulase ou alpha-glucosidase. Pour une détection directe, ce substrat peut être lié à une partie faisant office de marqueur, fluorescent ou chromogène. Pour une détection indirecte, le milieu de culture selon l'invention peut comporter en sus un indicateur de pH, sensible à la variation de pH induite par la consommation du substrat et révélant la croissance des microorganismes cibles. Ledit indicateur de pH peut être un chromophore ou un fluorophore.

Selon un premier mode de réalisation, le milieu sélectif objet de l'invention est utilisé en contrôle microbiologique alimentaire. Préférentiellement, il est utilisé pour la croissance et le dénombrement de *Staphylococcus aureus* dans les produits laitiers.

Selon un second mode de réalisation, le milieu sélectif objet de l'invention est utilisé en contrôle microbiologique de l'environnement. Par environnement, on entend des prélèvements d'air, des prélèvements d'eau, ou des prélèvements de surfaces. Parmi les prélèvements de surfaces, l'objet de l'invention peut trouver une application particulière dans la détection en milieu hospitalier de *Staphylococcus aureus,* parmi les staphylocoques à coagulase positive responsables d'infections nosocomiales.

Selon un dernier mode de réalisation, le milieu sélectif objet de l'invention est utilisé en analyse clinique pour détecter et/ou identifier, voire en outre dénombrer *Staphylococcus aureus*.

De façon avantageuse, le milieu sélectif objet de l'invention peut comporter en outre un marqueur de résistance, par exemple dans le cadre d'un test de résistance d'une souche de *Staphylococcus aureus* à la méthicilline.

Un autre objet de la présente invention concerne l'utilisation du milieu de culture selon l'invention pour la différenciation des bactéries *Staphylococcus aureus* par rapport aux staphylocoques à coagulase négative.

Un autre objet de la présente invention concerne l'utilisation du milieu de culture selon l'invention, pour détecter et/ou identifier, voire en outre dénombrer des bactéries *Staphylococcus aureus* dans un échantillon complexe.

Enfin, un dernier objet de la présente invention concerne un procédé de détection et/ou d'identification des bactéries *Staphylococcus aureus,* ledit procédé comprenant les étapes consistant à :
a) Ensemencer le milieu de culture selon l'invention, avec un échantillon susceptible de contenir des bactéries *Staphylococcus aureus;*
b) Mesurer une modification de la coloration dans le milieu de culture, cette modification correspondant à la croissance de bactéries *Staphylococcus aureus* dans ledit milieu de culture.

De façon avantageuse, le procédé selon l'invention comporte une étape intermédiaire a') consistant à mettre le milieu de culture ainsi ensemencé dans des conditions aptes à permettre la croissance des bactéries *Staphylococcus aureus.*

Selon un mode particulier de réalisation, le procédé selon l'invention peut comporter une étape supplémentaire de dénombrement de microorganismes cibles. Une telle étape de dénombrement est de préférence réalisée selon la méthode du Nombre le Plus Probable (NPP). Cette méthode est explicitée dans le brevet EP 1 105 457 au nom de la demanderesse.

De façon préférentielle, l'échantillon biologique est un échantillon clinique, alimentaire ou environnemental.

Il est à noter enfin que la mise en oeuvre des objets selon l'invention ne saurait être limitée à un seul type de support. En effet, tous les types de supports utilisés dans le domaine du diagnostic *in vitro* sont aptes à mettre mis en oeuvre : microplaques, microtubes, microcupules, capillaires, etc...

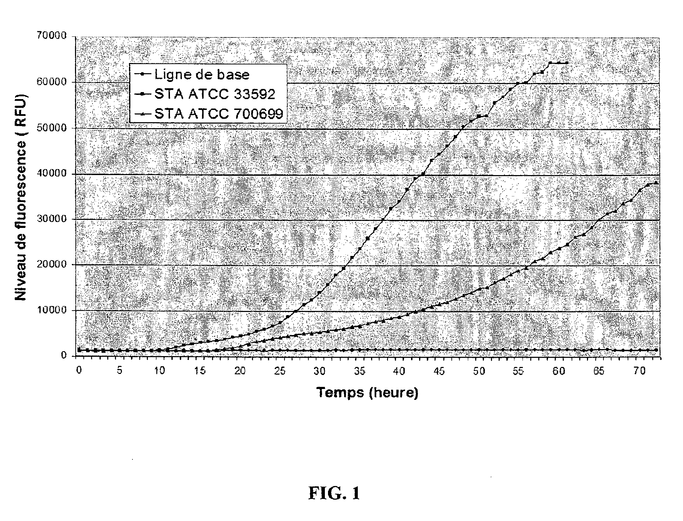

Les exemples suivants, en lien avec la figure 1 sont donnés à titre comparatif et n'ont aucun caractère limitatif.

La figure 1 représente la mesure de fluorescence au cours du temps, reflétant l'activité enzymatique PC-PLC (lécithinase) de deux souches de *Staphylococcus aureus*

### Exemple comparatif 1 : Etude de la corrélation entre l'activité PIPLC et la présence d'une coagulase chez Staphylococcus aureus - comparaison Système TEMPO® / milieu Baird-Parker + RPF (BP+RPF)

43 souches de *Staphylococcus aureus* et 20 souches de staphylocoques à coagulase négative ont été testées par ensemencement d'un milieu Baird-Parker + RPF (Ref. bioMérieux : 44003) et d'un milieu liquide dérivé, du milieu Ottaviani Agosti Agar (OAA), utilisé avec le système TEMPO® commercialisé par la demanderesse.

### Milieu dérivé de OAA :

Le milieu utilisé est de composition suivante :

| **Constituant** | **Concentration en g/l** |
|---|---|
| Extrait de levure | 5 |
| Biothione | 5 |
| Biosoyase | 5 |
| Biotrypcase | 5 |
| Pyruvate de Na | 2 |
| Glucose | 0,01 |
| Glycérophosphate de Mg | 1 |
| NaCl | 5 |
| LiCl | 5 |
| MgCl₂ | 1 |
| MgSO₄ | 0,5 |
| Tampon MOPS Acide | 14,7 |
| Tampon MOPS basique | 7 |
| 4-Methylumbelliferyl myo-inositol-1-phosphate | 0,4 |

- Tampon MOPS 0.1M à pH 6,70 (ajustement avec HCL 6N)
- Substrat : 4-Méthylumbelliferyl myo-inositol-1-phosphate, N-méthyl-morpholine salt, Biosynth, Réf. M-5717 Lot 20078/1)

Le milieu de culture décrit ci-dessus contient du 4-Methylumbelliferyl myo-inositol-1-phosphate auquel est associée une base nutritive pour permettre, simultanément à la croissance, une détection par apparition de fluorescence des *Staphylococcus aureus.*

### Ensemencement et incubation des cartes TEMPO® :

Les souches testées, à une concentration initiale de 10⁸ Unités Formant Colonie (UFC)/ml, sont diluées dans un bouillon tryptone sel, de façon à obtenir une concentration finale à 10³ UFC/ml.

50µl de cette suspension bactérienne sont ajoutés à 4 ml de milieu de culture. L'ensemble est chargé dans la carte TEMPO®, de sorte que la quantité en bactérie est de 50UFC/carte.

Les cartes TEMPO® sont incubées à 37°C pendant 24 heures.

### Ensemencement et incubation du milieu Baird-parker + RPF :

La suspension bactérienne à 10³ UFC/ml est également utilisée pour ensemencer le milieu Baird-parker + RPF à raison de 50µl.

Le milieu est incubé à 37°C pendant 24 heures.

### Résultats :

Les résultats obtenus avec la carte TEMPO® sont analysés par le système TEMPO®, conformément à la méthode du Nombre le Plus Probable (NPP). L'apparition d'une fluorescence est directement lié à l'activité catalytique de la PIPLC sur le 4-Méthylumbelliferyl myo-inositol-1-phosphate entraînant la libération de 4-Méthylumbelliferone fluorescente.

Les colonies obtenues sur le milieu Baird-parker + RPF sont classiquement dénombrées.

Les tableaux 1 et 2 ci-dessous regroupent les résultats obtenues par les deux méthodes :

**Tableau 1 : Corrélation entre l'activité PIPLC et la présence d'une coagulase chez Staphyloccocus aureus et dénombrements associés à chaque méthode (24h)**

| **Code** | **Espèce** | **Référence Souche** | **BP + RPF dénombrement / Coagulase (24h)** | **TEMPO® (24h)** | |
|---|---|---|---|---|---|
| | | | | **Activité PIPLC** | **NPP** |
| S1 | *S. aureus* | 0201058 | 47 / + | + | 30 |
| S2 | *S. aureus* | 9801012 | 86 / + | + | 112 |
| S3 | *S. aureus* | 9710057 | 24 / + | + | 44 |
| S4 | *S. aureus* | 9704027 | 42 / + | + | 40 |
| S5 | *S. aureus* | 8904051 | 32 / + | + | 28 |
| S6 | *S. aureus* | 8304011 | 50 / + | + | 40 |
| S7 | *S. aureus* | 8301044 | 99 / + | + | 112 |
| S8 | *S. aureus* | 7509008 | 68 / + | + | 56 |
| S9 | *S. aureus* | 8405022 | 62 / + | + | 72 |
| S10 | *S. aureus* | 0212024 | 57 / + | + | 56 |
| S11 | *S. aureus* | 0212017 | 35 / + | + | 44 |
| S12 | *S. aureus* | 0201059 | 45 / + | + | 21 |
| S13 | *S*. *aureus* | 9704026 | 75 / + | + | 128 |
| S14 | *S*. *aureus* | 8904052 | 49 / + | + | 12 |
| S15 | *S. aureus* | 9805029 | 35 / + | + | 19 |
| S16 | *S*. *aureus* | 8311065 | 61 / + | + | 39 |
| S17 | *S. aureus* | 7802083 | 45 / + | + | 26 |
| S18 | *S*. *aureus* | 9807062 | 20 / + | + | 28 |
| S19 | *S. aureus* | ATCC 12600 | 30 / + | + | 21 |
| S20 | *S. aureus* | CA5 130203 | 41 / + | + | 44 |
| S21 | *S*. *aureus* | 201058 | 400 / + | + | 680 |
| S22 | *S. aureus* | 0201060 | 47 / + | + | 28 |
| S23 | *S. aureus* | CA20 130203 | 80 / + | + | 92 |
| S24 | *S*. *aureus* | CA8 130203 | 49 / + | + | 44 |
| S25 | *S. aureus* | CA30 130203 | 24 / + | + | 16 |
| S26 | *S. aureus* | CA40 130203 | 43 / + | + | 44 |
| S27 | *S. aureus* | CA41 130203 | 75 / + | + | 96 |
| S28 | *S*. *aureus* | CA56 130203 | 73 / + | + | 52 |
| S29 | *S. aureus* | CA9 040303 | 53 / + | + | 56 |
| S30 | *S. aureus* | CA23 040303 | 85 / + | + | 48 |
| S31 | *S*. *aureus* | CA31 040303 | 49 / + | + | 44 |
| S32 | *S. aureus* | CA32 040303 | 51 / + | + | 84 |
| S33 | *S. aureus* | LCHA 4890 | 53 / + | + | 44 |
| S34 | *S*. *aureus* | Clermont | 33 / + | + | 36 |
| S35 | *S. aureus* | 57.10 | 40 / + | + | 44 |
| S36 | *S. aureus* | G52.61 | 48 / + | + | 23 |
| S37 | *S. aureus* | ATCC 6538P | 31 / + | + | 28 |
| S38 | *S. aureus* | IM 803 | 32 / + | + | 26 |
| S39 | *S*. *aureus* | P02-52 | 67 / + | + | 36 |
| S40 | *S. aureus* | P5678 | 11 / + | + | 14 |
| S41 | *S. aureus* | SP2 | 56 / + | + | 36 |
| S42 | *S. aureus* | 5271 | 35 / + | + | 30 |
| S43 | *S. aureus* | E1115 | 52 / + | + | 44 |

**Tableau 2 : Corrélation entre l'absence d'activité PIPLC et l'absence de coagulase chez Staphyloccocus spp et dénombrements associés à chaque méthode (40h)**

| **Code** | **Espèce** | **Référence Souche** | **BP + RPF dénombrement / Coagulase (40h)** | **TEMPO® (40h)** | |
|---|---|---|---|---|---|
| | | | | **Activité PIPLC** | **NPP** |
| S44 | *S. hyicus* | 83.01.033 | 38 / - | - | <1 |
| S45 | *S. epidermidis* | ATCC 12228 | 28 / - | + | 11 |
| S46 | *S. epidermidis* | 1566 | 26 / + | + | 26 |
| S47 | *S. epidermidis* | 0303001 | 30 / - | - | <1 |
| S48 | *S*. *xylosus* | ATCC 700404 | 7 / - | - | <1 |
| S49 | *S. schleiferi* | 322 | 34 / - | - | <1 |
| S50 | *S. schleiferi* | 4103 | 41 /- | - | <1 |
| S51 | *S*. *lugdunensis* | 228025 | 60 / - | - | <1 |
| S52 | *S. intermedius* | 217005 | 17 / - | - | <1 |
| S53 | *S. intermedius* | 2885 | 62 / - | - | <1 |
| S54 | *S. warneri* | N930256 | 5 / - | - | <1 |
| S55 | *S. warneri* | Huiller | 1 / - | - | <1 |
| S56 | *S. haemolyticus* | 128549 | 9 / - | - | <1 |
| S57 | *S. haemolyticus* | R202 | 14 / - | - | <1 |
| S58 | *S. saprophyticus* | 42.92 | 20 / - | - | <1 |
| S59 | *S. saprophyticus* | DiMarcheleo | 16 / - | - | <1 |
| S60 | *S. capriae* | 0512901 | 75 / - | - | <1 |
| S61 | *S. epidermidis* | 07.08.901 | 160 / - | - | <1 |
| S62 | *S*. *arlettae* | 92.03.376 | 159 / - | - | 3 |
| S63 | *S. lentus* | 85.05.027 | 1 / - | - | <1 |

- Toutes les souches de *Staphylococcus aureus* testées se sont développées sur le milieu Baird-parker + RPF après 24h d'incubation. Elles possèdent toutes une coagulase.
- Parmi les *staphylococcus* non *aureus,* seule la souche S46 montre la présence d'une coagulase. Une galerie API réalisée sur cette souche a démontré qu'il s'agit en réalité d'un *Staphylococcus aureus.*
- La souche S45 présente un phénotype coagulase - sur Baird-parker + RPF, mais on observe un signal positif avec TEMPO. Un isolement sur boite à montré une contamination de l'échantillon par des *Bacillus.*
- A 24h, deux souches de *Staphylococcus aureus* (S1 et S15) montrent une faible variation entre le signal positif et le bruit de fond. Ceci est en accord avec le diamètre peu important du halo d'éclaircissement autour de ces colonies sur Baird-parker + RPF. Une incubation prolongée (40 heures) a permis de confirmer un rapport signal sur bruit correct.
- On détecte l'activité PIPLC pour chacune des souches positives pour la coagulase sur Baird-parker + RPF. Les souches coagulase-négative n'induisent pas de signal positif pour l'activité PIPLC, sauf la S45.

Les résultats ainsi obtenus permettent donc de mettre clairement en évidence une corrélation entre l'activité coagulase de *Staphylococcus aureus* et l'activité PIPLC.

### Exemple comparatif 2 : Analyse de l'activité PIPLC chez Staphylococcus aureus en microplaques avec 2 substrats de PIPLC différents

19 souches de *Staphylococcus aureus* ATCC sont testées pour leur activité PIPLC en microplaques. Les essais sont réalisés avec deux substrats fluorogènes différents de PIPLC : le 4-méthylumbelliféryl-myo-inositol-1-phosphate et le 3-Chloro-7-hydroxy-4-méthylcoumarine myo-inositol-phosphate, ajoutés en concentrations différentes aux constituants du milieu dérivé du milieu OAA décrit dans l'exemple 1 .

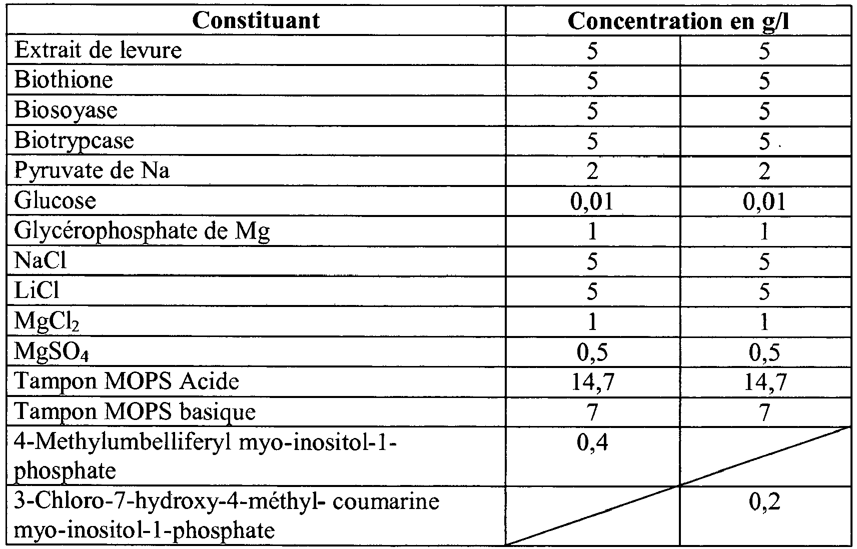

- 4-Méthylumbelliferyl myo-inositol-1-phosphate, N-méthyl-morpholine salt, Biosynth, (Réf. M-5717 Lot 20078/1)
- 3-Chloro-7-hydroxy-4-méthylcoumarine myo-inositol-1-phosphate, (PM 458.36)

### Synthèse du 3-Chloro-7-hydroxy-4-méthylcoumarine myo-inositol-1-phosphate

La synthèse de l'intermédiaire myo-inositol protégé est d'abord réalisée selon A. V. Rukavishnikov et al., Chem. Phys. Lipids, 89 (1997), 153-157.

Cet intermédiaire a ensuite été couplé avec le second intermédiaire préparé, à savoir la 3-chloro-7-hydroxy-4-méthyl-coumarine-diisopropylphosphoramidite. Le produit souhaité a été obtenu par une réaction de déméthylation du produit de couplage en présence de l'iodure de lithium, puis une déprotection de la partie inositol (T. O. Zaikova, et al., Bioconjugate Chem., 12 (2001), 307-313).

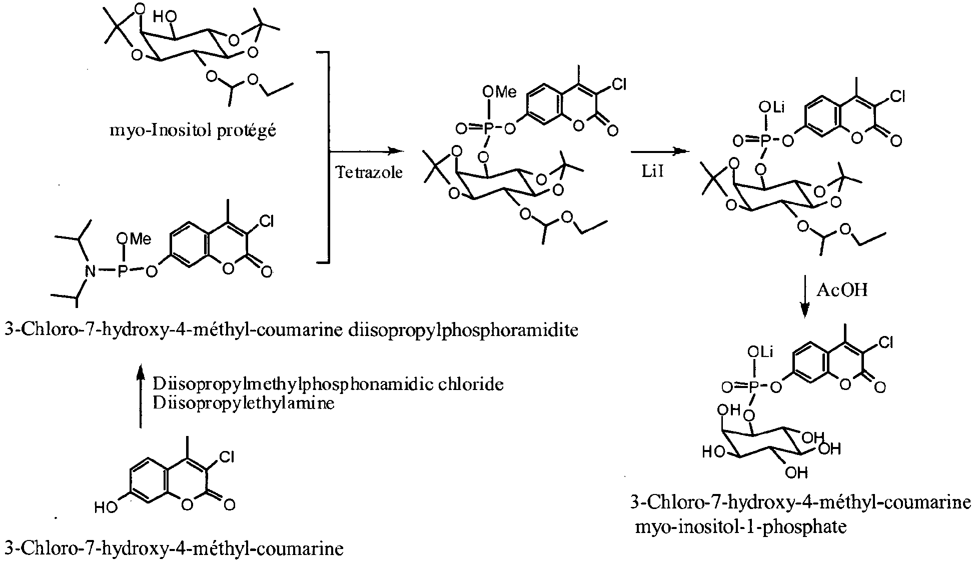

Les structures moléculaires du produit final ainsi que des intermédiaires ont été analysées en RMN.

### Ensemencement et incubation des microplaques :

Les souches testées, à une concentration initiale de 10⁸ (UFC)/ml, sont diluées dans un bouillon tryptone sel, de façon à obtenir une concentration finale à 10³ UFC/ml. 10µl de cette suspension bactérienne sont ajoutés à 200 µl de milieu de culture. Chaque souche a été incubée 24 heures à 37°C avec chacun des milieux dans le lecteur de microplaques commercialisé par la société TECAN sous la dénomination **GENios**^{™}.

### Vérification des inocula :

Afin de vérifier les inocula, 10 ml de chaque suspension bactérienne à 10³ UFC/ml servent à ensemencer un milieu Baird-Parker + RPF (Ref. bioMérieux : 44003).

### Résultats :

Les résultats obtenus en microplaques sont récapitulés dans le tableau 3 ci-dessous. Les signes + correspondent aux souches permettant la production d'une fluorescence au minimum 2 fois supérieure au bruit de fond. Des signes - correspondent à un signal émis par le milieu inférieur au bruit de fond.

**Tableau 3**

| Souches | Activité PIPLC | |
|---|---|---|
| | 4-Méthylumbelliféryl-myo-inositol-1-phosphate | 3-Chloro-7-hydroxy-4-méthyl-coumarine myo-inositol-1-phosphate |
| ATCC 9144 | + | + |
| ATCC 29213 | + | + |
| ATCC 12598 | + | + |
| ATCC 6538 | + | + |
| ATCC 43300 | + | + |
| ATCC 13150 | + | + |
| ATCC 49775 | - | - |
| ATCC 33862 | + | + |
| ATCC 51740 | + | + |
| CCM 6188 | - | + |
| ATCC 43866 | + | + |
| ATCC 33592 | + | + |
| ATCC 700699 | + | + |
| ATCC 700698 | + | + |
| ATCC 700789 | + | + |
| ATCC 700788 | + | + |
| ATCC 33591 | + | + |
| ATCC 51153 | + | + |
| ATCC 33593 | + | + |

Parmi les dix-neuf souches de *Staphylococcus aureus* ATCC testées, une seule ne présente aucun signal PIPLC (ATCC 49775), quel que soit le substrat utilisé. 17 présentent une activité PIPLC en présence de 4-Méthylumbelliféryl-myo-inositol-1-phosphate et 18 présentent une activité PIPLC en présence de 3-Chloro-7-hydroxy-4-méthyl-coumarine myo-inositol-1-phosphate.

L'activité PIPLC chez *Staphylococcus aureus* est ainsi bien confirmée.

Il est à noter par ailleurs que des mesures de l'activité PIPLC ont été réalisée, au total, sur 109 souches de *Staphylococcus aureus* (révélés coagulase positive sur milieu Baird-Parker + RPF) et 26 souches de staphylocoques à coagulase négative. Les résultats obtenus sont regroupés dans le tableau 4 ci-dessous :

**Tableau 4**

| **Types de souches** | **Nombre de souches testées** | **Présence d'une activité PIPLC** | **Absence d'une activité PIPLC** | **Pourcentage de souches présentant une activité PIPLC** |
|---|---|---|---|---|
| *Staphylococcus aureus* | 109 | 105 | 5 | **95.4** |
| Staphylocoques à coagulase négative | 26 | 0 | 26 | **0** |

Les résultats présentés ci-dessus confirment que la mesure de l'activité PIPLC est un paramètre très pertinent pour discriminer *Staphylococcus aureus* des staphylocoques à coagulase négative.

### Exemple comparatif 3: Analyse de l'activité PC-PLC (lécithinase) de Staphylococcus aureus en microplaques (Figure 1)

La dynamique de l'activité PC-PLC de deux souches de *Staphylococcus aureus* ATCC 33592 et ATCC 700699 a été évaluée en microplaque en présence du milieu ci-dessous. Une lecture d'apparition de la fluorescence dans chacun des différents puits de la microplaque a ensuite été effectuée en cinétique.

### 1. MILIEUX

Le milieu utilisé est de composition suivante, pH 7.2 :

| **Composés** | **Concentration en g/l** |
|---|---|
| Extrait de levure | 5 |
| Peptone pomme de terre | 2 |
| Rhodorsil | 0.4 |
| Sulfate de manganèse | 1 |
| Pyruvate de sodium | 2 |
| Glycérophosphate de magnésium | 1 |
| Histidine | 3 |
| NaCl | 5 |
| Tampon HEPES basique | 13.8 |
| Tampon HEPES acide | 11.92 |
| 4-Methylumbelliferyl choline-phosphate (4MU-CP) | 0.4 |
| Colistine | 0.01 |

La 4MU-CP est produite par la société Biosynth, sous la référence M-5528.

### 2. ESSAI

10 UFC de *Staphylococcus aureus* ATCC 33592 et ATCC 700699 ont été inoculées dans les puits de la microplaque en présence du milieu décrit ci-dessus. La microplaque est ensuite incubée à 37°C dans un lecteur de marque TECAN afin d'évaluer l'activité PC-PLC des deux souches de *Staphylococcus aureus* sous forme de cinétique d'hydrolyse du substrat 4MU-CP, i.e apparition de fluorescence.

### 3. RESULTATS & INTERPRETATION

Une mesure de l'apparition de la fluorescence consécutive à l'hydrolyse du substrat 4MU-CP a été effectuée sur une période d'incubation de 72h pour les deux souches de *Staphylococcus aureus* testées (STA ATCC 33592 et STA ATCC 700699) *versus* le témoin milieu non inoculé (ligne de base).

Les dynamiques d'activité PC-PLC des deux souches de *Staphylococcus aureus* étudiées sont représentées sur le graphique présenté en figure 1.

Ainsi, l'utilisation du substrat fluorogénique de PC-PLC, le 4MU-CP, permet la détection et la discrimination de *Staphylococcus aureus.*

## Revendications

1. Milieu de culture spécifique pour la croissance des bactéries *Staphylococcus aureus* et leur détection et/ou identification, ledit milieu étant **caractérisé par le fait qu'**il comporte au moins un substrat chromogène de phosphatidyl inositol phospholipase C (PIPLC) qui est le 4-Nitrophenyl myo-inositol-1-phosphate.

2. Utilisation du milieu de culture selon la revendication 1 pour la différenciation des bactéries *Staphylococcus aureus* par rapport aux staphylocoques à coagulase négative.

3. Utilisation du milieu de culture selon la revendication 1, pour détecter et/ou identifier, voire en outre dénombrer des bactéries *Staphylococcus aureus* dans un échantillon complexe.

4. Procédé de détection, et/ou d'identification des bactéries *Stophylococcus aureus,* ledit procédé comprenant les étapes consistant à :
a) Ensemencer le milieu de culture selon la revendication 1, avec un échantillon, susceptible de contenir des bactéries *Staphylococcus aureus ;*
b) Mesurer une modification du niveau de coloration dans le milieu de culture, ladite modification correspondant à la croissance de bactéries *Staphylococcus aureus* dans ledit milieu de culture.

5. Procédé selon la revendication 4 comportant une étape intermédiaire a') consistant à mettre le milieu de culture ainsi ensemencé dans des conditions aptes à permettre la croissance desdites bactéries.

6. Procédé selon la revendication 5, comportant une étape supplémentaire de dénombrement de *Staphylococcus aureus.*

7. Procédé selon l'une des revendications 4 à 6, dans lequel l'échantillon biologique est un échantillon clinique, alimentaire ou environnemental.

## Patentansprüche

1. Ein spezifisches Kulturmedium für das Wachstum von *Staphylococcus-aureus-*Bakterien und deren Nachweis und/oder Identifikation, wobei das Medium **dadurch gekennzeichnet ist, dass** es mindestens ein chromogenes Phosphatidylinositol-Phospholipase-C(PIPLC)-Substrat beinhaltet, das 4-Nitrophenyl-myo-inositol-1-phosphat ist.

2. Eine Verwendung des Kulturmediums gemäß Anspruch 1 zur Unterscheidung zwischen *Staphylococcus-aureus-*Bakterien und koagulasenegativen Staphylokokken.

3. Verwendung des Kulturmediums gemäß Anspruch 1 zum Nachweisen und/oder zum Identifizieren, darüber hinaus auch zum Zählen, von *Staphylococcus-aureus-*Bakterien in einer komplexen Probe.

4. Ein Verfahren zum Nachweis und/oder zur Identifikation von *Staphylococcus-aureus-*Bakterien, wobei das Verfahren die folgenden Schritte beinhaltet, bestehend aus:
a) Beimpfen des Kulturmediums gemäß Anspruch 1 mit einer Probe, von der vermutet wird, dass sie *Staphylococcus-auraus*-Bakterien enthält;
b) Messen einer Veränderung des Färbungsgrades in dem Kulturmedium, wobei die Veränderung dem Wachstum von *Staphylococcus-aureus-*Bakterien in dem Kulturmedium entspricht.

5. Verfahren gemäß Anspruch 4, beinhaltend einen Zwischenschritt a'), bestehend aus dem Bringen des derart beimpften Kulturmediums unter angemessene Bedingungen, die das Wachstum dieser Bakterien ermöglichen.

6. Verfahren gemäß Anspruch 5, beinhaltend einen zusätzlichen Schritt der Zählung von *Staphylococcus aureus.*

7. Verfahren gemäß einem der Ansprüche 4 bis 6, wobei die biologische Probe eine klinische, alimentäre oder Umwelt-Probe ist.

## Claims

1. Specific culture medium for growth of *Staphylococcus aureus* bacteria, and their detection and/or identification, said medium being **characterized in that** it comprises at least one chromogenic substrate of phosphatidylinositol phospholipase C (PIPLC) which is 4-nitrophenyl myo-inositol-1-phosphate.

2. Use of the culture medium according to claim 1, for differentiation of *Staphylococcus aureus* bacteria relative to coagulase-negative staphylococci.

3. Use of the culture medium according to claim 1, for detecting and/or identifying and in addition for counting *Staphylococcus aureus* bacteria in a complex sample.

4. Method of detecting and/or identifying *Staphylococcus aureus* bacteria, said method comprising the stages consisting of:
a) Seeding the culture medium according to claim 1, with a sample that may contain *Staphylococcus aureus* bacteria;
b) Measuring a change in the level of coloration in the culture medium, said change corresponding to the growth of *Staphylococcus aureus* bacteria in said culture medium.

5. Method according to claim 4, comprising an intermediate stage a') consisting of putting the culture medium thus seeded in conditions suitable for permitting the growth of said bacteria.

6. Method according to claim 5, comprising a supplementary stage of counting of *Staphylococcus aureus.*

7. Method according to one of claims 4 to 6, in which the biological sample is a clinical sample, food sample or environmental sample.
